# EUROPEAN PATENT SPECIFICATION

(11) **EP 1 235 890 B1**
(45) Date of publication and mention of the grant of the patent: **06.10.2004**
(21) Application number: 00974547.2
(22) Date of filing: 13.11.2000
(51) Int. Cl.: C11D 1/37, C11D 3/20, A61K 7/06, A61K 7/48, A61K 7/50, C11D 1/10

(54) **STABLE, HIGH GLYCEROL LIQUIDS COMPRISING N-ACYL AMINO ACIDS AND/OR SALTS**
STABILE FLÜSSIGKEITEN MIT ERHÖHTEM GLYCERINGEHALT, ENTHALTEND N-ACYLAMINOSÄURE UND/ODER DEREN SALZE
LIQUIDES STABLES A FORTE TENEUR EN GLYCEROL CONTENANT DES N-ACYL AMINOACIDES ET/OU LEURS SELS

(30) Priority: 06.12.1999 GB 9928822
(43) Date of publication of application: 04.09.2002
(73) Proprietor: UNILEVER PLC, London EC4P 4BQ (GB); UNILEVER N.V., 3013 AL Rotterdam (NL)
(72) Inventor: ARAI, Masaaki, Trumbull, CT 06611 (US); IWAMOTO, Yukiko Nippon Lever B.V. Innovation Ctr., Haga-gun Tochigi Tochigi 321-3325 (JP); KOBAYASHI, Mitsunobu Nippon L. B.V. Innovation Ctr, Haga-gun Tochigi Tochigi 321-3325 (JP); MISHINA, Yukie, Obihiro-shi Hokkaido 080-0044 (JP); TAKADA, Naohiko Nippon Lever B.V. Innovation Ctr., Haga-gun Tochigi Tochigi 321-3325 (JP)
(74) Representative: Mulder, Cornelis Willem Reinier, Dr.
(86) International application number: PCT/EP2000/011358
(87) International publication number: WO 2001/042409

(56) References cited:
- US-A- 4 749 515
- PATENT ABSTRACTS OF JAPAN vol. 018, no. 542 (C-1261), 17 October 1994 (1994-10-17) & JP 06 192064 A (LION CORP), 12 July 1994 (1994-07-12)
- PATENT ABSTRACTS OF JAPAN vol. 1998, no. 14, 31 December 1998 (1998-12-31) & JP 10 245323 A (LION CORP), 14 September 1998 (1998-09-14)

## Description

### Field of the Invention

The present invention relates to high content glycerol liquid compositions comprising N-acyl amino acids and/or salts thereof and also containing certain sulfosuccinic acid monoesters. More specifically, while high glycerin content has been found to destabilize such compositions, applicants have found that inclusion of dialkylene glycol stabilizes the compositions. Compositions of the invention are used primarily in skin cleansing, shower gel, and hair care compositions.

### Background of the Invention

Compositions containing N-acyl amino acids and/or salts and certain sulfosuccinic acid monoesters are beneficial to the skin. The use of both components together is broadly taught in U.S. Patent No. 4,749,515 to Miyamoto. Compositions of Miyamoto impart smoothness to the hair or skin after washing.

However, because of its skin moisturizing properties, it would be greatly beneficial to add glycerin to these or other compositions to help relieve dryness of skin. In the above compositions, however, it has been found that including large amounts of glycerin (e.g., about 10% and above, preferably 15% to 50% by wt., more preferably 20% to 45% by wt., more preferably 35% and above by wt.) led to instability and phase separation. While not wishing to be bound by theory, it is thought that this may be due to the high specific gravity of glycerin.

Unexpectedly, applicants have now found that if 0.5 to 25% by wt., preferably 1 to 15% by wt. of dialkylene glycol (e.g., dipropylene glycol) are used in the high content glycerin, N-acyl amino acid/sulfosuccinic acid monoester compositions, the compositions are stable (stability is maintained for at least 4 weeks at room temperature).

### Brief Summary of the Invention

The present invention relates to stable high content glycerol liquid detergent compositions. More particularly, the compositions comprise:
(1) 1-20%, preferably 5 to 20% by wt. of an N-acyl amino acid (e.g., N-acyl glycinate) or salts thereof;
(2) 0.5-15%, preferably 1-10%, by wt. of one or more sulfosuccinic acid monoesters represented by formula (I), (II),(III) or (IV); 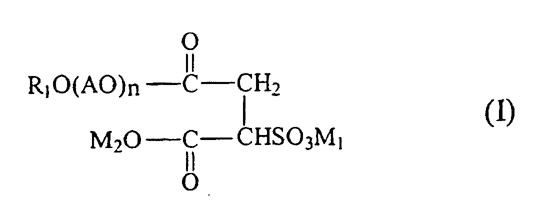 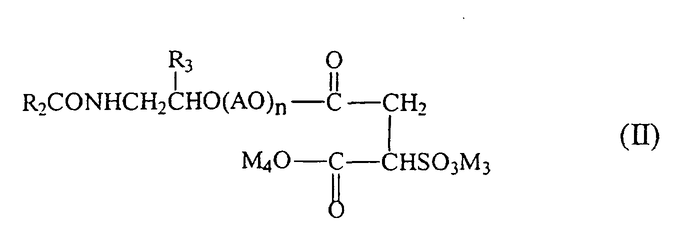 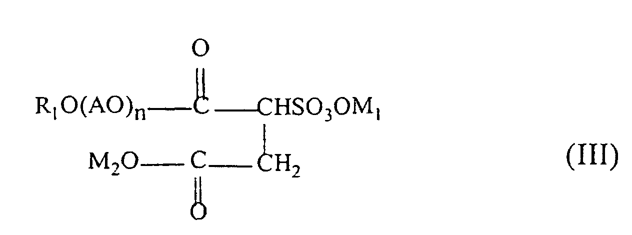 or 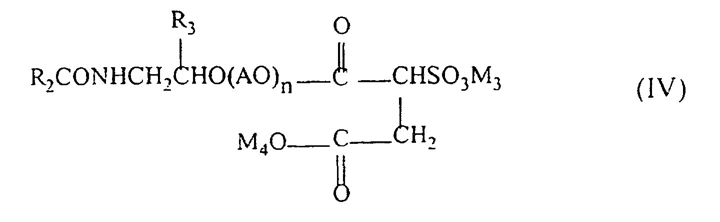 wherein each of M₁ to M₄ is H, NH₄, an alkali metal or a hydroxyalkyl substituted ammonium. R₁ and R₂ are each an alkyl or hydroxyalkyl group having about 8 to 20 carbon atoms, R₃ is H or CH₃, AO is an oxyalkylene group having 2 or 3 carbon atoms, and n represents an integer from 0 to 20;
(3) 10% and above, preferably 15% to 50% by wt., more preferably 20% to 45% by wt., more preferably 35% and above by wt. of glycerin; and
(4) 0 to 25% by wt., preferably 1 to 15% by wt. dialkylene glycol; and
(5) water to balance (e.g., 5 to 90%, preferably 10 to 80%, more preferably 20 to 75% water).

In a second embodiment, the invention relates to a method of enhancing the stability of high content glycerin compositions comprising N-acyl amino acids or salts thereof and sulfosuccinic acid monoesters as defined above by adding 1 to 20% by wt. of dialkylene glycol.

The pH of the compositions is about 5.5 to 8, preferably 6 to 7.5 and the viscosity is about 300 to 10,000 centistokes. Viscosity measurements were conducted using a Brookfield viscometer, Spindle No. 2 or 3, 12rpm, 30 seconds, at 25 degree C.

The stability of the composition is defined by the absence of separation after 4 weeks storage at room temperature.

### Detailed Description of the Invention

The present invention relates to stable, high content glycerol liquid detergent compositions used, for example, in skin cleansing, shower gel and/or shampoo compositions. More specifically, the compositions of the present invention comprise N-acyl amino acids and certain sulfosuccinic acid monoesters. Because of problems in maintaining stability, it has not been previously possible to make such compositions with high levels (i.e., equal to and above 10% up to 50% by wt. glycerol). Applicants have found, however, that addition of 0.5 to 25%, preferably 1 to 15% by wt. dialkylene glycol provides stability to such compositions. Compositions of the invention are described in greater detail below.

The compositions of the present invention contain 1 to 20% by wt., preferably 5 to 20% by wt. of N-acyl amino acid and salts thereof.

Acyl groups in the N-acyl amino acids and salts thereof which are suitable for the purposes of the present invention have 6 to 24 carbon atoms; for example, lauryl, myristyl, palmityl, or the like is included. The amino acids include glutamic acid, glycine and beta-alanine. The salts include alkali metal salts, hydroxyalkyl substituted ammonium salts and ammonium salts. The hydroxyalkyl substituted ammonium salts may preferably have 1 to 3 carbon atoms in the hydroxyalkyl group. N-acyl-N-alkyl amino acids are also included in the term "N-acyl amino acids" used herein. The alkyl groups in the N-acyl-N-alkyl amino acids may preferably have 1 to 3 carbon atoms and include methyl, ethyl, propyl, and isopropyl. These N-acyl amino acids and salts thereof may be used independently or as a combination of two or more.

Preferred N-acyl amino acids and salts thereof may include N-acyl amino acids such as N-lauroylglutamic acid, N-myristoylglutamic acid, N-palmitoyl-alpha-glutamic acid, N-myristoyl-beta-alanine, N-palmitoyl-beta-alanine, N-acyl N-alkyl amino acids such as N-lauroyl-N-ethylglycine, N-lauroyl-N-isopropylglycine, N-lauroylsarcosine, N-myristoylsarcosine, N-palmitoylsarcosine, N-lauroyl-N-methyl-beta-alanine, as well as their alkali metal salts and hydroxyalkyl-substituted ammonium salts.

The compositions of the present invention also comprise 0.5 to 15%, preferably 1-10% by wt. of a sulfosuccinic acid monoester represented by formulae (I), (II), (III) or (IV) or mixtures thereof as follows: 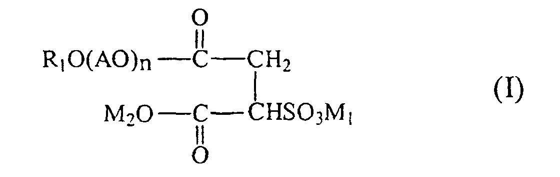 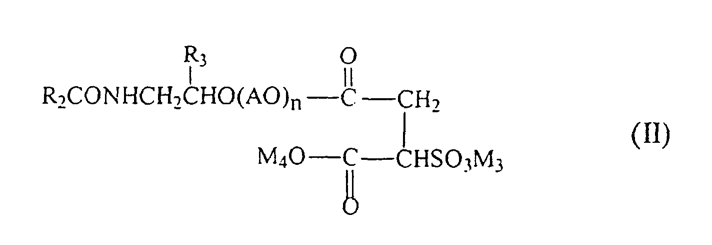 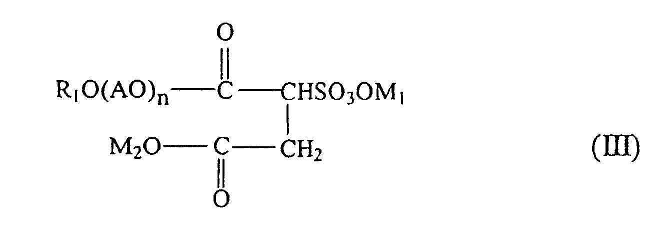 or

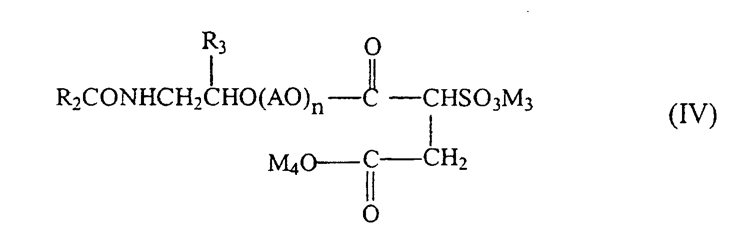

In these formulae, each of M₁ to M₄ may be selected from H, NH₄, alkali metals and hydroxyalkyl-substituted ammonium groups. M₁ and M₂, and M₃ and M₄ may be the same or different. Alkali metals may include lithium, sodium and potassium. The hydroxyalkyl substituted ammonium groups, which may preferably have 1 to 3 carbon atoms in the hydroxyalkyl group, may include monoethanol ammonium, diethanol ammonium, triethanol ammonium and methyl diethanol ammonium. Preferably M₁ to M₄ are hydrogen, sodium or triethanol ammonium.

R₁ and R₂ are each straight or branched alkyl or hydroxyalkyl groups having about 8 to 20 carbon atoms, for example, hexyl, decyl, hydroxydecyl, dodecyl, hydroxytetradecyl, tetradecyl or for nonadecyl. Any alkyl group having less than 8 or more than 20 carbon atoms may optionally be contained in the molecule, provided that the total number of carbon atoms in the sulfosuccinic acid monoester contained in the detergent composition of the present invention is in the range of from 8 to 20. This number of carbon atoms provides good foaming properties, whereas when the number of carbon atoms is either less than 8 or more than 20 less foaming is observed.

In the formulae (II) and (IV), R₃ is either H or CH₃.

In the formulae, AO represents an oxyalkylene group having 2 or 3, carbon atoms, that is, oxyethylene or oxypropylene group. Both oxyethylene and oxypropylene groups may be present in the molecule.

The letter n represents an integer from 0 to 20, preferably 0 to 10. If n is more than 20, the foaming properties of the resulting liquid detergent compositions may be poor.

The compounds represented by the formulae (I), (II), (III) and (IV) may be prepared by any known methods.

For example, the sulfosuccinic acid monoesters represented by the general formula (I) or (II) may be prepared by reacting an alkylene oxide adduct of a higher fatty acid with maleic anhydride to produce an ester of maleic anhydride and further reacting the ester of maleic anhydride with a sulfite.

Alternatively, the sulfosuccinic acid monoesters represented by the general formulae (II) or (IV) may be prepared by reacting a lower alcohol ester of a higher fatty acid with an alkanolamine, adding an alkylene oxide to the reaction product, further reacting the resulting addition product with maleic anhydride to produce an ester of maleic anhydride, and reacting the ester of maleic anhydride with a sulfite.

The compositions of the present invention also comprise about 10% and above, preferably 15% to 50% by wt, more preferably 20% to 45% by wt. of glycerin.

Finally, the compositions of the invention also comprise 1-20%, preferably 2-15% by wt. of a polyalkylene glycol, preferably dialkylene glycol.

Especially preferred are dialkylene glycol molecules such as, for example, diethylene or dipropylene glycol.

As noted above, it is the specific inclusion of such polyalkylene glycol molecules which is believed to stabilize the high content glycerin compositions of the present invention.

The compositions of the present invention may also contain many optional components as set forth below.

Specifically, in addition to the glycinate of the invention, compositions of the invention may contain 0.1 to 10% by wt. of an additional surfactant or surfactants selected from anionic, nonionic, amphoteric and cationic surfactants and mixtures thereof.

It is especially desired to contain at least 0.10 to 10% by wt. of an additional anionic surfactant.

Suitable anionic detergents include, but are not limited to, alkyl and alkylene carboxylates having 10 to 20 carbon atoms, alkyl ether carboxylates, fatty alcohol sulfates, fatty alcohol ether sulfates, alkylol amido-sulfates and sulfonates, fatty acid alkylol amido-polyglycol ether sulfates, alkane sulfonates and hydroxyalkane sulfonates, olefin sulfonates, acyl esters of isethionates, α-sulfo fatty acid esters, alkyl-benzene sulfonates, alkylphenol glycol ether sulfonates, sulfo-succinates, sulfosuccinic acid half and di-esters, fatty alcohol ether phosphates, protein fatty acid condensation products, alkyl monoglyceride sulfates and sulfonates, alkyl glyceride ether sulfonates, fatty acid methyl taurides, fatty acid sarcosinates, sulfo-ricinoleates. These compounds and mixtures thereof are used in the form of their water-soluble or water-dispersible salts, for example the sodium, potassium, magnesium, ammonium, mono-, di- and triethanolammonium salts and analogous alkylol ammonium salts.

A particularly preferred anionic surfactant is polyoxyalkylene alkylether sulfate which maybe used in an amount of,for example, 0.01 to 5% by wt.

Suitable nonionic surface-active agents include, for example, fatty alcohol ethoxylates (alkyl-polyethylene glycols), alkylphenol-polyethylene glycols, alkylation-polyethylene glycols, fatty amine ethoxylates (alkylamino-polyethylene glycols), fatty acid ethoxylates (acyl polyethylene glycols), polypropylene glycol ethoxylates (Pluronic (Registered Trademark)), fatty acid alkyl amides (fatty acid amino polyethylene glycols), saccharose esters, sorbitol esters, and polyglycol ether.

Suitable amphoteric surface-active agents to be added to the shampoos include N-alkyl-(3-amino dipropionates having 12 to 18 alkyl carbon atoms as alkali metal salts and mono-, di- and trialkylol-ammonium salts, N-acylamido-alkyl-N, N-dimethylacecobetaine, preferably N-acyl-amidopropyl-N,N-dimethylacetobetaine of 8 to 18 acyl carbon atoms, alkyl-dimethylsulfopropyl-betaine having 12 to 18 alkyl carbon atoms, amphoteric surfactants of the imidazoline type(Trademarks: Miranol Steinapon), preferably the sodium salt of 1-(β-carboxy-methyloxethyl)-1-(carboxymethyl)-2-lauryl-imidazolinium; amine oxides, for example alkyl-dimethyl-amine oxide of 12 to 18 alkyl carbon atoms, fatty acid amidoalkyl-dimethylamine oxide.

A particularly preferred amphoteric is cocoamido propyl betaine.

Among suitable cationics are included, for example quaternary ammonium salts, such as dialkyl-dimethyl-ammonium, chloride or bromide having 10 to 24, preferably 12 to 18 carbon atoms in the alkyl portion, alkyl-dimethyl-ethylammonium chloride or bromide having 10 to 24 alkyl carbon atoms, alkyl-trimethyl-ammonium chloride or bromide having 10 to 24 alkyl carbon atoms, preferably cetyl-trimethyl ammonium chloride or bromide, alkyl-trimethyl ammonium chloride or bromide having 10 to 22 alkyl carbon atoms, or alkyl-dimethyl-benzyl ammonium chloride or bromide having 10 to 24, preferably 12 to 18, carbon atoms in the alkyl portion.

In addition to the ingredients noted above, a variety of optional ingredients may be included in the compositions of the present invention.

Preferred optional ingredients are free fatty acids, i.e., C₁₀-C₂₄, preferably C₁₂ to C₁₈ straight chained, preferably saturated fatty acids. Examples include lauric and/or myristic acid. These should be used in an amount from about 0.1 to 10%, preferably 2 to 8% by wt. While not wishing to be bound by theory, the fatty acids are believed to enhance stability.

The liquid personal cleansing compositions of the present invention may optionally also include water-dispersible, gel-forming polymers. The polymer is preferably a anionic, nonionic, cationic or hydrophobically modified polymer, selected from cationic polysaccharides of the cationic guar gum class with molecular weights of 1,000 to 3,000,000, anionic, cationic and nonionic homopolymers derived from acrylic and/or methacrylic acid, anionic, cationic and nonionic cellulose resins; cationic copolymers of dimethyldialkyl ammonium chloride and acrylic acid; cationic homopolymers of dimethyldialkyl ammonium chloride; cationic polyalkylene and ethoxypolyalkylene imines polyethylene glycol with molecular weights of 10,000 to 4,000,000; and mixtures thereof. Preferably, the polymer is selected from Sodium Polyacrylate, Hydroxy Ethyl Cellulose, Cetyl Hydroxy Ethyl Cellulose, and Polyquaternium 10.

The polymer is preferably included in the compositions of the present invention at a level of from about 0.1 parts to 1 part, more preferably 0.1 parts to 0.5 parts. The polymers improve the sensory feel of the lipid on skin in addition to providing product stabilization. The improved sensory feel results from reduced tackiness and greasiness and improved smoothness. In an especially. preferred embodiment a mixture of polymers is used, for example, those polymers preferred for product stabilization, some are preferred for improved sensory feel and/or those preferred polymers for improved sensory feel. Preferred polymers for improved sensory feel are selected from polyethylene glycol, hydroxypropyl guar, guar hydroxypropyl trimonium chloride, polyquaternary 3, 5, 6, 7, 10, 11 and 24 and mixtures thereof.

The balance of the composition (generally between about 10 to 70%, preferably 20 to 60% by wt.) is water.

A variety of additional ingredients may be incorporated into the compositions of the present invention. These materials include, but not limited to, liquid appearance aids, salts and their hydrates and other "filler materials" are listed in U.s. Patent No. 5,340,492 to Kacher et *al.,* and U.S. Patent No, 4, 919, 934 to Deckner *et al.*.

Other non-limiting examples of these additional ingredients include vitamins and derivatives thereof (e.g., ascorbic acid, vitamin E, tocopherol acetate, sunscreens, thickening agents (e.g., polyol alkoxy ester, available as Crothix from Croda at levels up to 2% and xanthan gum at levels up to 2%), preservatives for maintaining the anti-microbial integrity of the compositions, anti-acne medicaments (resorcinol, salicylic acid), antioxidants, skin soothing and healing agents such as aloe vera extract, allantoin, chelators (e.g., EDTA and hydroxy ethan diphosphoric acid) and sequestrants; and agents suitable for aesthetic purposes such as fragrances, essential oils, skin sensates, pigments, pearlescent agents (e.g., mica and titanium dioxide), additives to impart a draggy rinse feel (e.g., fumed silica), additives to enhance deposition (e.g., maleated soybean oil at levels up to 3%), lakes, colorings (e.g., clove oil, menthol, camphor, eucalyptus oil and eugenol).

In a second embodiment, the invention relates to a method of enhancing the stability of high glycerin content compositions containing N-acyl amino acids or salts thereof and defined sulfosuccinic acid monoesters by adding 1 to 20% of polyalkylene glycol, preferably dialkylene glycol, as noted above.

Compositions of the present invention have a pH of 5.5 to 8, preferably 6 to 7.5.

Compositions of the present invention have a viscosity of 300-10,000 centistokes measured using a Brookfield Viscometer, Spindle 2 and 3 at 12 rpm for 30 seconds at 25°C.

The compositions of the present invention exhibit no precipitation or phase separation after 4 weeks storage at room temperature (i.e., about 25°C).

### EXAMPLES

### Example 1 and 2

The following are two examples of the compositions of the invention.

| | **Example 1** | **Example 2** |
|---|---|---|
| Sodium N-cocoyl glycinate(SCG) | 10% | 10% |
| Disodium laurylether sulfosuccinate(DSLES) | 2% | 2% |
| Disodium lauryl sulfosuccinate(DSLS) | 2% | 2% |
| Lauric acid | 3% | 3% |
| Myristic acid | 2% | 2% |
| Potassium hydroxide | 0.67% | 0.67% |
| Sodium laurylether sulfate(SLES) | 0.88% | 0.88% |
| Cocamidopropyl betaine (CAPB) | 0.12% | 0.12% |
| Ethylene glycol distearate(EGDS) | 3% | 3% |
| Dipropylene glycol(DPG) | 3% | 3% |
| Glycerin | 37% | 20% |
| Amphoteric polymer | 0.3% | 0.3% |
| Alkyl acrylate polymer | 0.2% | 0.5% |
| Tetrasodiumedetate tetrahydrate (EDTA) | 0.05% | 0.05% |
| Dibutylhydroxtoluene(BHT) | 0.05% | 0.05% |
| Preservative | 0.3% | 0.3% |
| Perfume | 0.7% | 0.7% |
| Water | To 100% | To 100% |

The compositions noted above were prepared as follows:

### Processing

1) A mixture of mostly glycerin and SCG was heated to 75°-80°C until becoming quite fluid (mixture-1);
2) A mixture of fatty acids, preservative, DPG, BHT and SLES was heated to 75°-80°C until becoming fluid (mixture-2);
3) Mixture-2 was added to mixture-1 with agitation(mixture-3);
4) A mixture of potassium hydroxide and water was heated to 75°-80°C (mixture-4);
5) Mixture-4 was added to mixture-3 with agitation (mixture-5);
6) CAPB, DSLES, DSLS and EGDS were separately added to mixture-5 with agitation (mixture-6);
7) Mixture-6 was cooled to 45°C;
8) Water and EDTA were mixed and then added to mixture-6 (mixture-7);
9) Water and amphoteric polymer were mixed and then added to mixture-7 (mixture-8);
10) Glycerin and alkyl acryl polymer were mixed and then added to mixture-8 (mixture-9);
11) Perfume was added to mixture-9 and cooled to 35°C.

### Example 3

In a composition containing DPG and glycerin the following data was observed in a composition as set forth in Example 1 (levels of glycerin were offset by water).

| DPG | Glycerin | RT stability |
|---|---|---|
| 0% | 40% | Separation; no good |
| 3% | 37% | Acceptable level; good |
| 5% | 35% | Acceptable level; good |
| 10% | 30% | Acceptable level; slightly good |
| 0% | 30% | Separation; no good |

The data above clearly shows that defined levels of dialkylene glycol enhance stability of high glycerin content compositions containing N-acyl amino acid and sulfosuccinic acid monoesters while, in their absence, phase separation occurs.

## Claims

1. A stable, high glycerol liquid detergent composition comprising:
(a) 1-20% by wt. N-acyl amino acid or salt of such acid;
(b) 0.5-15% by wt. of one or more sulfosuccinic acid monoesters reprsented by Formula (I) or (II); 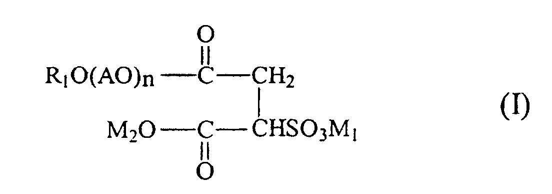 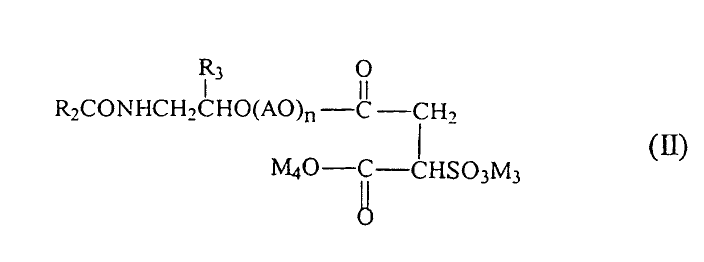 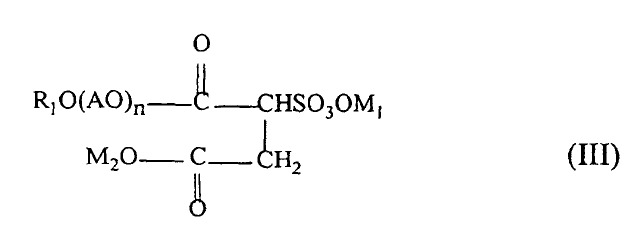 or 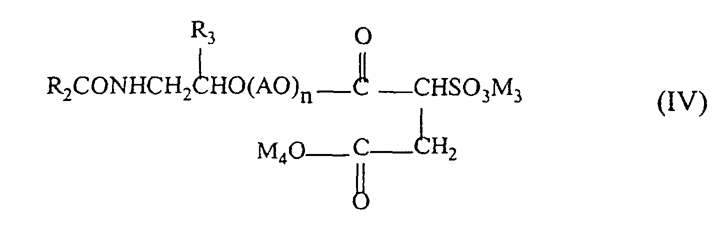 wherein each of M₁ to M₄ is H, NH₄, an alkali metal or a hydroxyalkyl substituted ammonium. R₁ and R₂ are each an alkyl or hydroxyalkyl group having about 8 to 20 carbon atoms, R₃ is H or CH₃, AO is an oxyalkylene group having 2 or 3 carbon atoms, and n represents an integer from 0 to 20;
(c) 10% and above by wt. of glycerin;
(d) 1-20%, by wt. of dialkylene glycol; and
(e) balance water.

2. A composition according to claim 1, wherein the composition additionally comprises 0.01-10% of a surfactant selected from anionic, nonionic, amphoteric and cationic surfactants and mixtures thereof.

3. A compositions according to claim 2, wherein the amphoteric surfactant is a betaine.

4. A composition according to any one of claims 1 to 3, wherein the composition additionally comprises 0.01 to 10% free fatty acid.

5. A composition according to any of the preceding claims, wherein the composition has a pH' of 5.5 to 8.

6. A composition according to claim 1, wherein the composition has a viscosity of 300 to 10,000 centistokes.

7. A composition according to claim 1, wherein the compositions has no precipitation and no phase separation after 4 weeks at room temperature.

## Patentansprüche

1. Stabile flüssige Detergens-Zusammensetzung mit hohem Glycerin-Gehalt, die
(a) 1-20 Gew.-% einer N-Acyl-Aminosäure oder eines Salzes einer derartigen Säure;
(b) 0,5-15 Gew.-% eines oder mehrerer Sulfobernsteinsäuremonoester(s), der/die durch die Formel (I) oder (II) 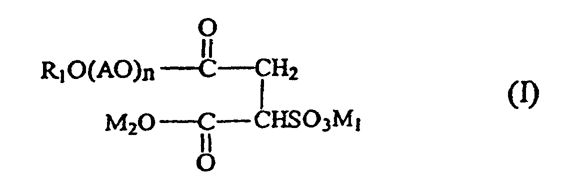 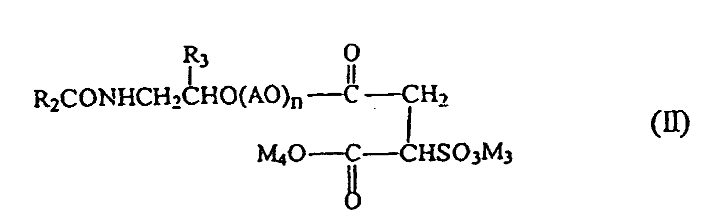 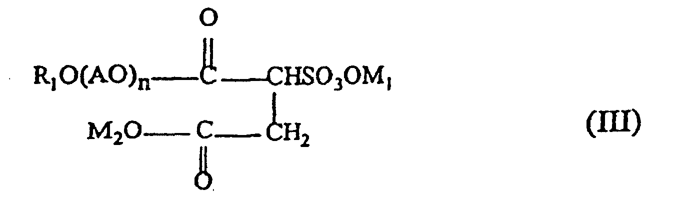 oder 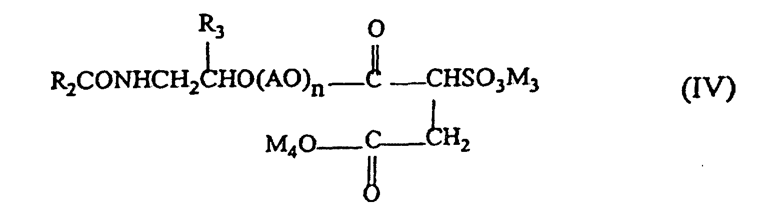 dargestellt ist/sind, wobei jedes von M₁ bis M₄ H, NH₄, ein Alkalimetall oder Hydroxyalkyl-substituiertes Ammonium ist, R₁ und R₂ jeweils eine Alkyl- oder Hydroxyalkyl-Gruppe mit etwa 8 bis 10 Kohlenstoffatomen sind, R₃ H oder CH₃ ist, AO eine Oxyalkylengruppe mit 2 oder 3 Kohlenstoffatomen ist und n eine ganze Zahl von 0 bis 20 darstellt;
(c) 10 Gew.-% und darüber Glycerin;
(d) 1-20 Gew.-% Dialkylenglycol; und
(e) der Rest Wasser umfasst.

2. Zusammensetzung nach Anspruch 1, wobei die Zusammensetzung zusätzlich 0.01-10 % eines oberflächenaktiven Mittels, das aus anionischen, nicht-ionischen, amphoteren und kationischen oberflächenaktiven Mitteln und Gemischen davon ausgewählt ist, umfasst.

3. Zusammensetzung nach Anspruch 2, wobei das amphotere oberflächenaktive Mittel Betain ist.

4. Zusammensetzung nach einem der Ansprüche 1 bis 3, wobei die Zusammensetzung zusätzlich 0,01 to 10 % freie Fettsäure umfasst.

5. Zusammensetzung nach einem der vorhergehenden Ansprüche, wobei die Zusammensetzung einen pH-Wert von 5,5 bis 8 aufweist.

6. Zusammensetzung nach Anspruch 1, wobei die Zusammensetzung eine Viskosität von 300 bis 10.000 Centistokes aufweist.

7. Zusammensetzung nach Anspruch 1, wobei die Zusammensetzung nach 4 Wochen bei Raumtemperatur keine Präzipitatbildung und keine Phasentrennung aufweist.

## Revendications

1. Composition détergente liquide stable à haute teneur en glycérol, comprenant :
(a) 1 à 20 % en poids d'acide N-acyl aminé ou un sel d'un tel acide ;
(b) 0,5 à 15 % en poids d'un ou plusieurs monoesters d'acide sulfosuccinique représenté par la formule (I) ou (II) ; 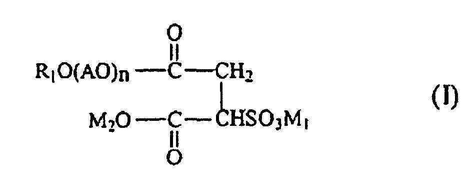 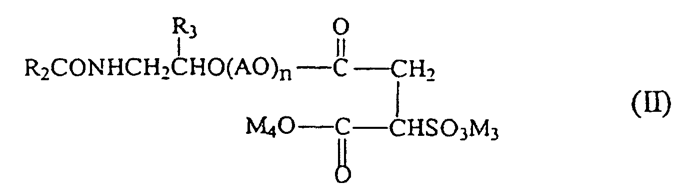 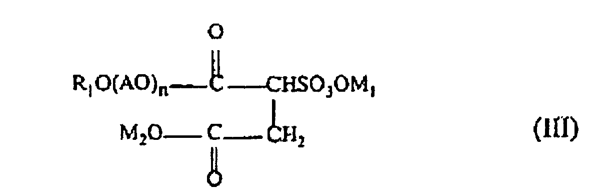 ou 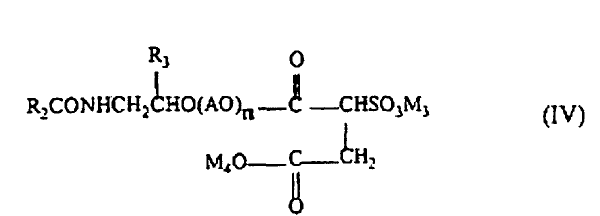 dans lesquelles chacun des M₁ à M₄ est H, NH₄, un métal alcalin ou un ammonium substitué par hydroxyalkyle. R₁ et R₂ sont chacun un groupe alkyle ou hydroxyalkyle ayant environ 8 à 20 atomes de carbone, R₃ est H ou CH₃, AO est un groupe oxyalkylène ayant 2 ou 3 atomes de carbone, et n représente un entier de 0 à 20 ;
(c) 10 % en poids et plus de glycérine ;
(d) 1 à 20 % en poids d'un dialkylène glycol ; et
(e) le reste en eau

2. Composition selon la revendication 1, dans laquelle la composition comprend de manière additionnelle 0,01 à 10 % d'un agent tensioactif choisi parmi les agents tensioactifs anioniques, amphotères et cationiques et les mélanges de ceux-ci.

3. Composition selon la revendication 2, dans laquelle l'agent tensioactif amphotère est une bétaïne.

4. Composition selon l'une quelconque des revendications 1 à 3, dans laquelle la composition comprend de manière additionnelle 0,01 à 10 % d'un acide gras libre.

5. Composition selon l'une quelconque des revendications précédentes, dans laquelle la composition a un pH de 5,5 à 8.

6. Composition selon la revendication 1, dans laquelle la composition a une viscosité de 300 à 10 000 centistokes.

7. Composition selon la revendication 1, dans laquelle la composition ne présente aucune précipitation et aucune séparation de phase après 4 semaines à température ambiante.
